(19) Europäisches Patentamt
European Patent Office
Office européen des brevets

(11) **EP 2 848 281 B1**

(12) **EUROPÄISCHE PATENTSCHRIFT**

(45) Veröffentlichungstag und Bekanntmachung des Hinweises auf die Patenterteilung:
**24.02.2016  Patentblatt 2016/08**

(51) Int Cl.:
*A61N 1/05* (2006.01)        *A61N 1/08* (2006.01)

(21) Anmeldenummer: **14180608.3**

(22) Anmeldetag: **12.08.2014**

(54) **Elektrodenvorrichtung für ein medizinisches Implantat und medizinisches Implantat mit einer Elektrodenvorrichtung**

Electrode device for a medical implant, and medical implant comprising an electrode device

Dispositif d'électrodes pour un implant médical et implant médical doté d'un dispositif d'électrodes

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.09.2013  US 201361878060 P**

(43) Veröffentlichungstag der Anmeldung:
**18.03.2015  Patentblatt 2015/12**

(73) Patentinhaber: **BIOTRONIK SE & Co. KG**
**12359 Berlin (DE)**

(72) Erfinder:
• **Büssing, Heinrich**
  **10317 Berlin (DE)**
• **Rump, Jens**
  **12049 Berlin (DE)**

(74) Vertreter: **Galander, Marcus et al**
**Biotronik SE & Co. KG**
**Corporate Intellectual Properties**
**Woermannkehre 1**
**12359 Berlin (DE)**

(56) Entgegenhaltungen:
CN-A- 102 826 842        US-A1- 2008 116 997
US-A1- 2009 040 131      US-A1- 2012 296 350

• **DAVID SOURIOU ET AL: "Antenna miniaturization and nanoferrite magneto-dielectric materials", 2010 14TH INTERNATIONAL SYMPOSIUM ON ANTENNA TECHNOLOGY AND APPLIED ELECTROMAGNETICS & THE AMERICAN ELECTROMAGNETICS CONFERENCE, 1. Juli 2010 (2010-07-01), Seiten 1-4, XP055161370, DOI: 10.1109/ANTEM.2010.5552533 ISBN: 978-1-42-445049-7**

**Beschreibung**

Die Erfindung betrifft eine Elektrodenvorrichtung für ein medizinisches Implantat und ein medizinisches Implantat mit einer Elektrodenvorrichtung nach den Oberbegriffen der unabhängigen Ansprüche.

Medizinische Implantate wie implantierbare Defibrillatoren, Herzschrittmacher etc. stimulieren den Herzmuskel mit Hilfe elektrischer Impulse, die von einem batteriebetriebenen Aggregat über eine Elektrode zum Herzen geführt werden.

Träger von Herzschrittmachern sind bei Einwirken starker statischer und wechselnder Magnetfelder von mehr als einem Tesla, wie sie etwa in diagnostischen Geräten wie Magnet-Resonanz-Tomographen zur Anwendung kommen, strengen Beschränkungen unterworfen, um eine Gefährdung durch Wechselwirkung der Magnetfelder mit Komponenten des Herzschrittmachers zu vermeiden. Herzschrittmacher und implantierbare Defibrillatoren etc. können durch die starken Magnetfelder beschädigt werden oder durch Wechselwirkungen mit den Magnetfeldern zur Schädigung des Patienten führen. So können sich Kontaktflächen von implantierten Elektroden erwärmen, magnetische Teile des Implantats könnten sich im Magnetfeld bewegen oder es kann eine Funktionsstörung der elektrischen Steuerung des Implantats eintreten. Träger von Herzschrittmachern dürfen daher, wenn überhaupt, nur unter bestimmten Voraussetzungen in einem Magnet-Resonanz-Tomographen untersucht werden.

Bei Magnet-Resonanz-Tomographen wird durch Überlagerung eines statischen Magnetfelds $B_0$ mit einem magnetischen Wechselfeld im Körper des Patienten eine Präzessionsbewegung der Kernspins der Wassermoleküle angeregt, die mit der Larmorfrequenz erfolgt. Die Larmorfrequenz hängt von der Stärke des statischen Magnetfeldes $B_0$ und vom betrachteten Teilchen, z.B. Protonen, ab und beträgt für Protonen bei einem Tesla 42,58 MHz. Das hochfrequentes Zusatzfeld, das quer zum statischen Magnetfeld orientiert ist und dessen Frequenz mit der Larmorfrequenz in Resonanz ist, lenkt alle Kerne phasensynchron aus ihrer aktuellen Lage zum statischen Feld $B_0$ aus. Signale bei der Relaxation der Kerne, bzw. des Kernspins, können erfasst und zur hochgenauen Bilddarstellung des Gewebes verwendet werden. Die Larmorfrequenz liegt im UKW-Bereich und stört die elektronischen Komponenten von medizinischen Implantaten.

Aus US2008/0116997 sind Bandfilter zur Verwendung in implantierbaren Stimulationsleitungen bekannt, die die Ausbreitung von MRI bedingten Frequenzen verhindern. US 2009/0040131 beschreibt magnetodielektrische Materialien zur Herstellung von Antennen, Filter und anderen Bauteilen zur Anwendung im Bereich der HF-Technologie. US2012/0296350 beschreibt eine Oberflächenbeschichtung für implantierbare medizinische Geräte basierend auf Metamaterialien um deren Sichtbarkeit im MR Bild zu erhöhen. Die Beschichtung kann zu frequenzselektiv aktiven Mustern bearbeitet werden, um die Ausbreitung von Wirbelströmen zu reduzieren. Die Metamaterialien umfassen ein nicht-magnetisches Metall als Basis (z.B. Ti,Ta, MP35N alloy), in welche leitfähige Metalle wie Au,Ag,Cu,Al,Pt, Pd diffundiert werden.

Der Erfindung liegt die Aufgabe zugrunde, eine Elektrodenvorrichtung für ein medizinisches Implantat zu schaffen, welche eine verbesserte Toleranz hinsichtlich der Wirkung von starken Magnetfeldern aufweist.

Eine weitere Aufgabe besteht darin, ein medizinisches Implantat mit einer entsprechenden Elektrodenvorrichtung zu schaffen.

Die Aufgaben werden erfindungsgemäß durch die Merkmale des unabhängigen Anspruchs 1 gelöst.

Günstige Ausgestaltungen und Vorteile der Erfindung ergeben sich aus den weiteren Ansprüchen und der Beschreibung.

Die Erfindung geht aus von einer Elektrodenvorrichtung für ein medizinisches Implantat, wobei die Elektrodenvorrichtung ein distales Ende und ein proximales Ende aufweist, zwischen denen sich eine elektrische Übertragungsleitung erstreckt.

Es wird vorgeschlagen, dass in der Übertragungsleitung wenigstens ein adaptives Element vorgesehen ist, das magnetodielektrisches Material aufweist, das bei Einwirken eines Magnetfeldes seine elektrischen und/oder magnetischen Eigenschaften ändert.

Dabei ist am proximalen Ende ein Aggregat angeordnet, in dem Energie zur Erzeugung der Impulse zur Stimulierung des Herzens bereitgestellt wird. Das distale Ende ist in der Nähe des Herzens oder direkt am Herzen angeordnet. Vorteilhaft kann die Elektronik des medizinischen Implantats auf die durch das jeweilige statische Magnetfeld ($B_0$-Feld) hervorgerufenen Larmorfrequenzen verschiedener Magnet-Resonanz-Geräte angepasst werden.

Magnetodielektrische Materialien sind so genannte Multiferroika und weisen eine relative Dielektrizitätskonstante ($\varepsilon_r$) und eine magnetische Permeabilität ($\mu_r$) oberhalb von eins auf. Charakteristisch für diese Materialien ist, dass beim Anlegen eines Magnetfeldes sich die Dielektrizitätskonstante $\varepsilon_r$ ändert, während sich beim Anlegen eines elektrischen Feldes die magnetischen Eigenschaften ($\mu_r$) ändern. Vertreter solcher magnetodielektrischen Materialien sind beispielsweise $BaMnF_4$, $BiMnO_3$, $LiCoPO_4$, $CoCr_2O_4$, $ZnMn_2O_4$, $La_2NiMnO_6$, $GeCo_2O_4$, $Mn_3O_4$, $(Tb, Dy, Ho)Mn_2O_5$, und $(La_{2/3}Ca_{1/3})MnO_3$. Insbesondere Verbindungen wie $La_2NiMnO_6$ und $(La_{2/3}Ca_{1/3})MnO_3$ weisen dabei bereits bei Raumtemperatur einen großen Effekt bei Magnetfeldern um 1 Tesla auf.

Eine Bedingung für eine erfolgreiche Anwendung ist, dass sich die Dielektrizitätskonstante Er bei dem Übergang von 1.5 Tesla zu 3 Tesla auf ¼ der Dielektrizitätskonstante Er ändert da gilt: f=1/sqrt(LC) f die Frequenz sich also quadratisch ändert.

Vorteilhaft kann das adaptive Element im Bereich des distalen Endes der Übertragungsstrecke angeordnet sein.

Gemäß einer günstigen Ausgestaltung kann das adaptive Element eine elektrische Spule umfassen. Vorteilhaft kann die Spule wenigstens bereichsweise mit magnetodielektrischem Material umwickelt sein. Alternativ oder zusätzlich kann die Spule wenigstens bereichsweise magnetodielektrisches Material umgeben. Hierdurch kann sich die elektrische Resonanzlänge der elektrischen Spule in definierter Weise abhängig von einem einwirkenden, insbesondere statischen Magnetfeld $B_0$ ändern. Hierdurch wird die Spule eine regelbare Spule, deren elektrische Eigenschaften in bekannter Weise durch das statische Magnetfeld $B_0$ veränderbar sind. Die Kapazität des Bauteils in Abhängigkeit des Magnetfeldes muss dazu beispielsweise folgender Beziehung gehorchen: $C = 1/\sqrt{B_0 \gamma L}$

Mit B0 der Magnetische Flussdichte, L der Induktivität des Bauteils und $\gamma$ dem gyromagnetisches Verhältnis.

Gemäß einer günstigen Ausgestaltung kann das adaptive Element eine elektrische Kapazität umfassen. Beim Einwirken eines Magnetfelds kann sich der Wert der elektrischen Kapazität in definierter Weise abhängig von einem einwirkenden, insbesondere statischen Magnetfeld $B_0$ ändern. Hierdurch wird die Kapazität eine regelbare Kapazität, deren elektrische Eigenschaften in bekannter Weise durch das statische Magnetfeld $B_0$ veränderbar sind. Die Kapazität kann als diskretes Bauteil ausgeführt sein. Die Resonanzfrequenz des Filters $f_{res} = 1/\sqrt{C L}$ sollte jeweils der Lamorfrequenz $f_{Lamor} = B_0\gamma$ entsprechen oder zumindest soweit an diese angenähert sein, dass eine ausreichende Dämpfung der eingekoppelten RF-Felder erreicht wird.

Gemäß einer günstigen Ausgestaltung kann das adaptive Element Bestandteil eines LC-Sperrfilters sein. Der Sperrfilter kann sich automatisch an das einwirkende Magnetfeld $B_0$ anpassen.

Gemäß einem weiteren Aspekt der Erfindung wird eine Verwendung einer erfindungsgemäßen Elektrodenvorrichtung in einem Herzschrittmacher vorgeschlagen.

Gemäß einem weiteren Aspekt der Erfindung wird eine Verwendung einer erfindungsgemäßen Elektrodenvorrichtung in einem implantierbaren Defibrillator vorgeschlagen.

Gemäß einem weiteren Aspekt der Erfindung wird ein medizinisches Implantat zur Implantation in den menschlichen oder tierischen Körper mit einer erfindungsgemäßen Elektrodenvorrichtung vorgeschlagen, bei dem in einer Übertragungsleitung zwischen einem Aggregat und einer Elektrodenspitze wenigstens ein adaptives Element vorgesehen ist, das magnetodielektrisches Material aufweist, welches bei Einwirken eines Magnetfeldes seine elektrischen und/oder magnetischen Eigenschaften ändert.

Die Erfindung ist nachfolgend beispielhaft, anhand von in Zeichnungen dargestellten Ausführungsbeispielen, näher erläutert. Es zeigen in schematischer Darstellung:

Fig. 1 eine erste Ausgestaltung eines distalen Endes einer Elektrodenvorrichtung nach der Erfindung;

Fig. 2 eine weitere Ausgestaltung eines distalen Endes einer Elektrodenvorrichtung nach der Erfindung; und

Fig. 3 eine Darstellung eines medizinischen Implantats mit einer weiteren Ausgestaltung einer Elektrodenvorrichtung.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

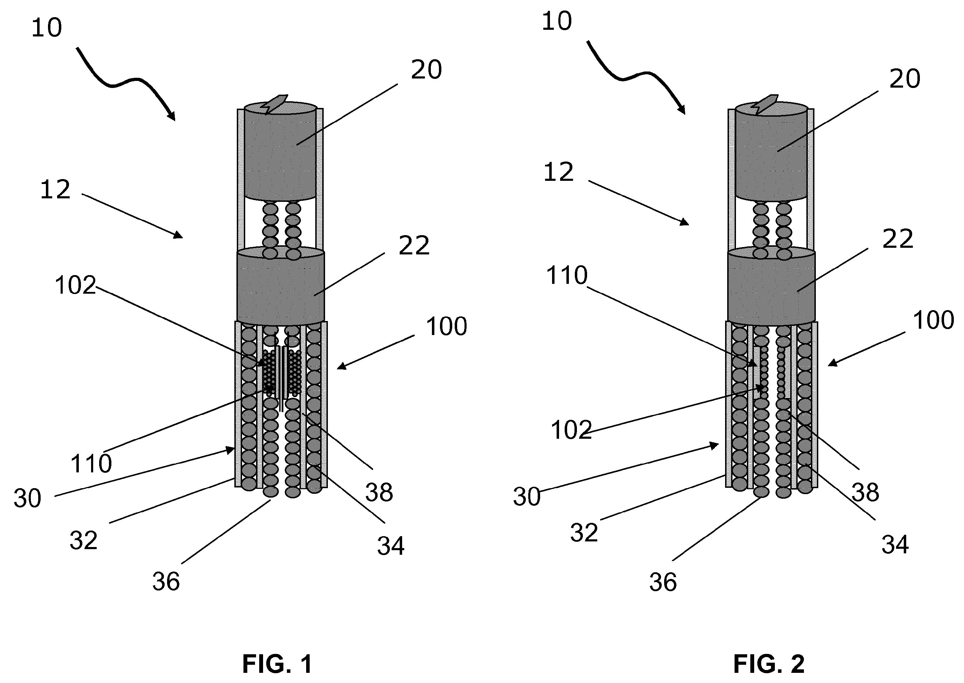

Die Figuren 1 und 2 zeigen zur Erläuterung der Erfindung jeweils Ausgestaltungen einer Elektrodenvorrichtung 10 für ein medizinisches Implantat. Das medizinische Implantat kann beispielsweise ein Herzschrittmacher, ein implantierbarer Defibrillator, ein Neurostimulator, ein Monitoringimplantat oder dergleichen sein. Möglich ist auch die Anwendung einer Elektrodenvorrichtung 10 als temporär implantierbare oder implantierbare Elektrode in Verbindung mit einem Externen Gerät wie einem externen Herzschrittmacher oder dergleichen. Auch ist eine Implemntierung der Elektrodenvorrichtung 10 in einen Katheter möglich.

Die Elektrodenvorrichtung 10 weist ein distales Ende 12 auf, das in den Figuren 1 und 2 als Detail dargestellt ist. An einem proximalen Ende (nicht dargestellt) ist ein Aggregat angeordnet, das eine Energiequelle, typischerweise eine Batterie, aufweist, sowie eine Elektronikeinheit zum Steuern und/oder Regeln von elektrischen Impulsen, die am distalen Ende 12 über eine Elektrodenspitze 20 an ein Einsatzgebiet, beispielsweise an einen Herzmuskel, abzugeben sind.

Zwischen dem proximalen Ende und dem distalen Ende 12 erstreckt sich eine elektrische Übertragungsleitung 30, innerhalb deren elektrisch isolierenden Hülle 32 sich zwei konzentrisch angeordnete Spulen 36 (innere Spule) und 34 (äußere Spule) von einem Ende zum anderen Ende erstrecken. Zwischen innerer Spule 36 und äußerer Spule 34 kann ebenfalls eine elektrische Isolierung 38 angeordnet sein. Am distalen Ende 12 endet die äußere Spule 34 an einem ringförmigen Endelement 22, durch welches die innere Spule 36 bis zur Elektrodenspitze 20 ragt, die mit dem Einsatzgebiet in unmittelbaren Kontakt kommen kann.

In der Übertragungsleitung 30 ist im Bereich des distalen Endes 12 ein adaptives Element 100 vorgesehen, das magnetodielektrisches Material 110 aufweist, welches bei Einwirken eines Magnetfeldes seine elektrischen und/oder magnetischen Eigenschaften ändert. Die Positionierung des adaptiven Elementes 100 am proximalen Ende ist möglich und schützt das aktive Implantat gegen Hochfrequenzströme, die Erwärmung am distalen Ende wird so aber nicht optimal unterdrückt.

In den Figuren 1 und 2 ist das adaptive Element 100 in der Übertragungsleitung 30 als elektrische Spule 102 ausgebildet, wobei in Figur 1 im Innern der Spule 102 magnetodielektrisches Material 110 angeordnet ist. So kann die Spule 102 um einen Kern aus magnetodielektrischem Material gewickelt sein kann. In Figur 2 umgibt eine Schicht magnetodielektrischen Materials 110 die Spule 102. Beispielsweise ist die Spule 102 mit einer oder mehreren Lagen magnetodielektrischen Materials 102 umwickelt. Das adaptive Element 100 unterbricht die innere Spule 36. Verändert sich das adaptive Element 100 in seinen elektrischen Eigenschaften, wenn ein starkes äußeres Magnetfeld $B_0$ einwirkt, so ändert sich die Resonanzlänge der Übertragungsleitung 30 und insbesondere der inneren Spule 36 entsprechend.

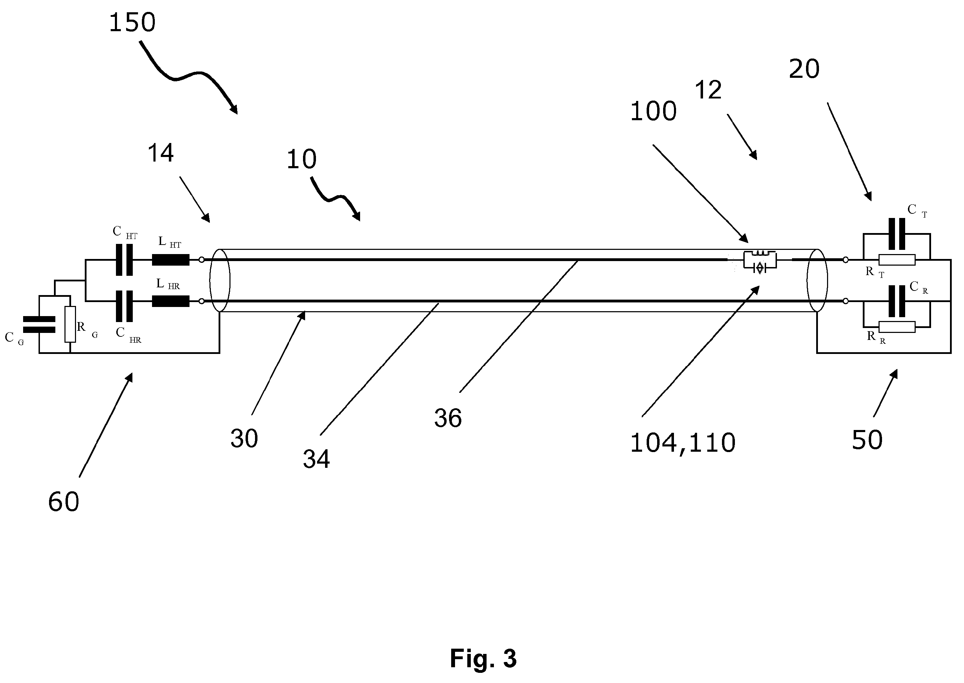

Figur 3 illustriert eine Ausgestaltung eines medizinische Implantats 150, beispielsweise eines Herzschrittmachers oder implantierbaren Defibrillators, mit einer vorteilhaften Elektrodenvorrichtung 10.

Am proximalen Ende 14 der geschirmt ausgeführten Übertragungsleitung 30 der Elektrodenvorrichtung 10 ist eine übliche Steuerelektronik 60 mit diversen Kapazitäten, Spulen und elektrischen Widerständen angeordnet, welche die Abgabe von elektrischen Impulsen aus der Elektrodenspitze 20 steuert oder regelt. Bekannte und übliche weitere Elemente wie Batterie oder Mikroprozessor sind nicht dargestellt.

Am distalen Ende 12 der Übertragungsleitung 30 ist das adaptive Element 100 angeordnet, das eine elektrische Kapazität 104 umfasst. Das adaptive Elements 100 ist Bestandteil eines LC-Sperrfilters.

Die elektrische Kapazität 104 weist magnetodielektrisches Material 110 auf, welches bei Einwirken eines Magnetfeldes seine elektrischen und/oder magnetischen Eigenschaften ändert. Beispielsweise kann das magnetodielektrische Material 110 zwischen gegensinnig gepolten Kondensatorelektroden angeordnet sein. Die Kapazität 104 kann als separates Bauteil ausgeführt sein.

**Patentansprüche**

1. Elektrodenvorrichtung (10) für ein medizinisches Implantat (150), wobei die Elektrodenvorrichtung (10) ein distales Ende (12) und ein proximales Ende (14) aufweist, zwischen denen sich eine elektrische Übertragungsleitung (30) erstreckt, **dadurch gekennzeichnet, dass** in der Übertragungsleitung (30) wenigstens ein adaptives Element (100) vorgesehen ist, das magnetodielektrisches Material (110) aufweist, das bei Einwirken eines Magnetfeldes seine elektrischen und/oder magnetischen Eigenschaften ändert.

2. Elektrodenvorrichtung nach Anspruch 1, **dadurch gekennzeichnet, dass** das adaptive Element (100) im Bereich des distalen Endes (12) der Übertragungsstrecke (30) angeordnet ist.

3. Elektrodenvorrichtung nach Anspruch 1 oder 2, **dadurch gekennzeichnet, dass** das adaptive Element (100) eine elektrische Spule (102) umfasst.

4. Elektrodenvorrichtung nach Anspruch 3, **dadurch gekennzeichnet, dass** die Spule (102) wenigstens bereichsweise mit magnetodielektrischem Material (110) umwickelt ist.

5. Elektrodenvorrichtung nach Anspruch 3 oder 4, **dadurch gekennzeichnet, dass** die Spule (102) wenigstens bereichsweise magnetodielektrisches Material (110) umgibt.

6. Elektrodenvorrichtung nach einem der vorhergehenden Ansprüche, **dadurch gekennzeichnet, dass** das adaptive Element (100) eine elektrische Kapazität (104) umfasst.

7. Elektrodenvorrichtung nach einem der vorhergehenden Ansprüche, **dadurch gekennzeichnet, dass** das adaptive Element (100) Bestandteil eines LC-Sperrfilters ist.

8. Verwendung einer Elektrodenvorrichtung (100) nach einem der vorhergehenden Ansprüche in einem Herzschrittmacher (150).

9. Verwendung einer Elektrodenvorrichtung (100) nach einem der vorhergehenden Ansprüche in einem Defibrillator.

10. Medizinisches Implantat (150) zur Implantation in den menschlichen oder tierischen Körper mit einer Elektrodenvorrichtung (100) nach einem der vorhergehenden Ansprüche, **dadurch gekennzeichnet, dass** in einer Übertragungsleitung (30) zwischen einem Aggregat (60) und einer Elektrodenspitze (20) wenigstens ein adaptives Element (100) vorgesehen ist, das magnetodielektrisches Material (110) aufweist, welches bei Einwirken eines Magnetfeldes seine elektrischen und/oder magnetischen Eigenschaften ändert.

**Claims**

1. An electrode device (10) for a medical implant (150), wherein the electrode device (10) has a distal end (12) and a proximal end (14), between which an electric transmission line (30) extends, **characterised in that** at least one adaptive element (100) is provided in the transmission line (30) and comprises magnetodielectric material (110), which changes its electric and/or magnetic properties under the action of a magnetic field.

2. The electrode device according to Claim 1, **characterised in that** the adaptive element (100) is arranged in the region of the distal end (12) of the transmission path (30).

3. The electrode device according to Claim 1 or 2, **characterised in that** the adaptive element (100) comprises an electric coil (102).

4. The electrode device according to Claim 3, **characterised in that** magnetodielectric material (110) is wound around the coil (102) at least in regions.

5. The electrode device according to Claim 3 or 4, **characterised in that** the coil (1) surrounds magnetodielectric material (110) at least in regions.

6. The electrode device according to one of the preceding claims, **characterised in that** the adaptive element (100) comprises an electric capacitor (104).

7. The electrode device according to one of the preceding claims, **characterised in that** the adaptive element (110) is part of an LC band-stop filter.

8. Use of an electrode device (100) according to one of the preceding claims in a cardiac pacemaker (150).

9. Use of an electrode device (100) according to one of the preceding claims in a defibrillator.

10. A medical implant (150) for implantation in a human or animal body, comprising an electrode device (100) according to one of the preceding claims, **characterised in that** at least one adaptive element (100) is provided in a transmission line (30) between a unit (60) and an electrode tip (20) and comprises magnetodielectric material (110), which changes its electric and/or magnetic properties under the action of a magnetic field.

**Revendications**

1. Dispositif d'électrode (10) pour un implant médical (150), dans lequel le dispositif d'électrode (10) présente une extrémité distale (12) et une extrémité proximale (14) entre lesquelles s'étend une ligne électrique de transmission (30), **caractérisé en ce qu'**au moins un élément d'adaptation (100), qui présente un matériau magnéto-diélectrique (110), est prévu dans la ligne électrique de transmission (30) qui modifie ses propriétés électriques et/ou magnétiques lors de l'influence d'un champ magnétique.

2. Dispositif d'électrode selon la revendication 1, **caractérisé en ce que** l'élément d'adaptation (100) est disposé dans la zone de l'extrémité distale (12) du trajet de transmission (30).

3. Dispositif d'électrode selon les revendications 1 ou 2, **caractérisé en ce que** l'élément d'adaptation (100) comprend une bobine électrique (102).

4. Dispositif d'électrode selon la revendication 3, **caractérisé en ce que** la bobine (102) est enroulée au moins localement avec le matériau magnéto-diélectrique (110).

5. Dispositif d'électrode selon les revendications 3 ou 4, **caractérisé en ce que** la bobine (102) entoure au moins localement le matériau magnéto-diélectrique (110).

6. Dispositif d'électrode selon l'une des revendications précédentes, **caractérisé en ce que** l'élément d'adaptation (100) comprend une capacité électrique (104).

7. Dispositif d'électrode selon l'une des revendications précédentes, **caractérisé en ce que** l'élément d'adaptation (100) est un constituant d'un filtre de réjection LC.

8. Utilisation d'un dispositif d'électrode (100) selon l'une des revendications précédentes dans un stimulateur cardiaque (150).

9. Utilisation d'un dispositif d'électrode (100) selon l'une des revendications précédentes dans un défibrillateur.

10. Implant médical (150) destiné à l'implantation dans le corps humain ou animal avec un dispositif d'électrode (100) selon l'une des revendications précédentes, **caractérisé en ce qu'**au moins un élément d'adaptation (100), qui présente un matériau magnéto-diélectrique (110), lequel modifie ses propriétés électriques et/ou magnétiques lors de l'influence d'un champ magnétique, est prévu dans une ligne de transmission (30) entre un système composite (60) et une pointe d'électrode (20).

**FIG. 1**

**FIG. 2**

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20080116997 A
- US 20090040131 A
- US 20120296350 A